# EUROPÄISCHE PATENTANMELDUNG

(11) **EP 1 110 567 A1**
(43) Veröffentlichungstag der Anmeldung: **27.06.2001**
(21) Anmeldenummer: 00127760.7
(22) Anmeldetag: 19.12.2000
(51) Int. Cl.: A61M 5/14, A61M 5/172

(54) **Infusionsvorrichtung mit mehreren Infusionspumpen**

(30) Priorität: 24.12.1999 DE 29922736 U
(71) Anmelder: B. BRAUN MELSUNGEN AG, 34212 Melsungen (DE)
(72) Erfinder: Heitmeier, Rolf, Dipl.-Ing., 34225 Baunatal (DE)
(74) Vertreter: Selting, Günther, Dipl.-Ing.

(57) **Zusammenfassung**

Die Infusionsvorrichtung weist ein zentrales Steuergerät (10) und zahlreiche Infusionsgeräte (12) auf, die mit dem Steuergerät (10) über jeweils einen Datenkanal (15) verbunden sind. Jedes Infusionsgerät (12) ist mit einer unverwechselbaren Identnummer versehen. Bei Anschluss eines Infusionsgerätes (12) an das Steuergerät (10) wird die Identnummer abgefragt und das Steuergerät prüft, ob sie bereits vorliegt. Liegt sie nicht vor, sendet das Steuergerät (10) die invertierte Identnummer an das Infusionsgerät (12) zurück. Dieses akzeptiert Befehle von dem Steuergerät erst, wenn bekannt wurde, dass das zurückgesandte Signal der Identnummer entspricht.

## Beschreibung

Die Erfindung betrifft eine Infusionsvorrichtung mit einem aus mehreren Infusionspumpen bestehenden Pumpenregime mit einem zentralen Steuergerät, das mit den einzelnen Infusionspumpen über Datenkanäle kommuniziert.

Komplexer werdende Medikationen von Intensivpatienten machen es erforderlich, dass eine zentrale Aufbereitung der Medikationsdaten erfolgt. Die bisher als Dokumentationssysteme bezeichneten technischen Lösungen erlauben aus sicherheitstechnischen Überlegungen eine Fernsteuerung der Infusionspumpen. Wesentliche Ursache für diese Einschränkung ist, dass programmierbare Systeme in einkanaliger Struktur keine Sicherheit gegen einen ersten Fehler ermöglichen. Aus diesem Grund wurden zweikanalige Strukturen geschaffen. Damit war das Problem unbemerkter Rechenfehler und unbemerkter Speicherveränderungen gelöst, jedoch wird damit noch nicht die notwendige Gesamtsicherheit erreicht, die zur Vernetzung zweier sicherheitstechnisch einwandfreier Systeme erforderlich ist. Dies wird dann deutlich, wenn man ein Infusionsregime, das üblicherweise aus 9 Pumpen oder anderen medizinisch technischen Geräten bestehen kann, unter sicherheitstechnischen Aspekten zu einem Gesamtsystem zusammenfassen will. Voraussetzung für eine derartige Lösung sind folgende:
- eindeutige Identifikation jedes einzelnen Infusionsgerätes
- Verhinderung der Verfälschung von Daten durch die physikalisch einkanalige Übertragung
- Vermeidung falscher Zuordnung (Adressierung) von Infusionsgeräten
- Erkennung des Entfernens eines Infusionsgeräts aus dem Infusionsregime
- Parallelbedienung automatisch/manuell
- aktionsabhängige Infusion, d.h. Bolusgabe für die Dauer eines Tastendrucks.

Der Erfindung liegt die Aufgabe zugrunde, eine Infusionsvorrichtung mit zentralem Steuergerät und mehreren Infusionsgeräten zu schaffen, welche die Sicherheitsvoraussetzungen in hohem Maße erfüllt.

Die Lösung dieser Aufgabe erfolgt erfindungsgemäß mit den im Anspruch 1 angegebenen Merkmalen. Hiernach enthält jedes Infusionsgerät eine unverwechselbare Identnummer. Die Infusionsvorrichtung führt nach Anschluss einer Pumpe einen Identifikationsprozess durch, dessen zentrales Element die einzigartige Identnummer des Infusionsgerätes ist. Das Steuergerät wird aufgefordert, vor einer Fernsteuerung das neu entdeckte Infusionsgerät nach seiner Identnummer abzufragen. Daraufhin sendet das Infusionsgerät die Identnummer an das Steuergerät. Dieses überprüft die Identnummer darauf, ob sie bereits vorliegt. Ist dies nicht der Fall, wird die Identnummer identisch oder in modifizierter Form an das Infusionsgerät zurückgeschickt. Erst wenn dort das zurückgeschickte Signal als korrekt erkannt wurde, akzeptiert das Infusionsgerät Fernsteuerbefehle vom Steuergerät. Das Steuergerät stellt anhand der Überprüfung der Identnummer sicher, dass die Identnummer in dem betreffenden Infusionsregime nur einmal bekannt ist. Hierdurch wird verhindert, dass unbeabsichtigt mehrfach vorkommende Identnummern zur Anwendung kommen. Andererseits erkennt das Infusionsgerät an dem zurückgeschickten Signal, dass das Steuergerät eine Überprüfung der Identnummer mit possitivem Ergebnis beendet hat, und akzeptiert erst dann die Fernsteuerbefehle des Steuergerätes. Durch die Erfindung wird sichergestellt, dass ein datentechnisch adressierbares Infusionsgerät in dem Regime nur einmal vorhanden ist und eindeutig identifiziert werden kann. Es wird vermieden, dass zwei Infusionsgeräte mit gleicher Identnummer vorhanden sind. Dies würde zur parallelen Beeinflussung beider Infusionsgeräte durch den Steuerrechner führen und hätte eine unbeabsichtigte Überdosierung zur Folge.

Vorzugsweise besteht das der Identnummer entsprechende, vom Steuergerät zurückgeschickte Signal aus der invertierten Identnummer. Dies bedeutet, dass die 0 Bits in 1 Bits umgewandelt werden und die 1 Bits in 0 Bits. Damit kann mit einfachen Logikmitteln ein Vergleich zwischen der Identnummer und der modifizierten Identnummer erfolgen. Selbstverständlich sind auch komplexere Modifizierungen der Identnummer möglich.

Das Problem der Verfälschung von Daten durch die physikalisch einkanalige Übertragung wird gemäss einer bevorzugten Weiterbildung der Erfindung dadurch gelöst, dass das Steuergerät und der Steuerteil des Infusionsgeräts zweikanalig ausgeführt sind und dass jeweils ein Kanal das zu übertragende Datum zur Verfügung stellt, während der andere ein dem Datum entsprechendes Sicherungswort generiert. Hierbei werden Datensicherungsverfahren, bei denen zu einzelnen Codeworten jeweils ein vom Inhalt des Codewortes abhängiges Sicherungswort generiert wird, auf die Zweikanaltechnik übertragen, wobei der eine Kanal das Codewort (Datum) überträgt und der andere das Sicherungswort. Codewort und Sicherungswort werden über den einzigen Datenkanal übertragen. Die zweikanalige Struktur von Steuergerät und Infusionsgerät wird zur Datensicherung benutzt. Während der erste Kanal das zu übertragende Datum zur Verfügung stellt, generiert der zweite Kanal unabhängig vom ersten das zugehörige Sicherungswort. Das Sicherungswort besteht beispielsweise aus einem CRC-Polynom. Es wird sichergestellt, dass nicht zufällig ein Sicherungswort vom Daten bereitstellenden Kanal angehängt oder erzeugt wird.

Die Daten, die vom Steuergerät erzeugt werden, müssen das jeweilige Infusionsgerät verwechslungsfrei erreichen. Dies wird durch Nutzung der Identnummer realisiert. Die mit einem Sicherungswort gesicherten Daten werden auch durch Zufügen der Identnummer markiert. Das Infusionsgerät überprüft die so markierten Daten auf Übereinstimmung mit der eigenen Identnummer. Nur bei Gleichheit werden die Daten akzeptiert.

Das Herausnehmen eines Infusionsgerätes aus dem Regime oder die unbeabsichtigte Unterbrechung der Verbindung zwischen Infusionsgerät und Steuergerät kann zu Überdosierungen führen, wenn beispielsweise die Rate des jeweiligen Infusionsgeräts durch einen Steueralgorithmus im Steuergerät kontinuierlich verändert wird. Das Infusionsgerät erwartet innerhalb einer vorgegebenen Fehlertoleranzzeit einen gültigen Kommunikationsablauf mit dem Steuergerät. Bei Ausbleiben des Kommunikationsablaufs wird die Medikation gestoppt. Die Medikation geht dann in den für einen Patienten sicheren Betriebszustand über.

Das Steuergerät ist vorzugsweise derart ausgebildet, dass es zwei Betriebsarten ausführen kann, wobei in einem Betriebsmodus "Fernsteuerung" Bedienungseingriffe am Infusionsgerät akzeptiert und Verstellungen am Steuergerät ignoriert werden, und in einem Betriebsmodus "Fernregelung" Bedienungseingriffe an dem Infusionsgerät ignoriert und Verstellungen am Steuergerät akzeptiert werden. Dadurch wird das Problem der Parallelbedienung gelöst, das sich dadurch ergibt, das Steuergerät und Infusionsgerät jeweils für sich bedient werden können. So ist der Fall möglich, dass das Steuergerät aufgrund einer zeitvarianten Ratensteuerung die Infusionsrate auf den Wert XXX stellt, während der Anwender an dem Infusionsgerät den Wert YYY einstellt. Die Erfindung sieht zur Lösung dieses Problems die beiden genannten Betriebsarten vor.

Gemäß einer Weiterbildung der Erfindung ermöglicht die Infusionsvorrichtung eine aktionsabhängige Infusion. Hierunter kann man die Situation verstehen, dass für die Dauer eines Tastendruckes auf eine Taste oder auf die Benutzeroberfläche des Steuergerätes eine definierte Förderrate an das Infusionsgerät zu übertragen ist bzw. dass das Infusionsgerät für die Dauer des Tastendrucks eine Rate XXX auszuführen hat. Erfindungsgemäß erfolgt die Übertragung des Tastendrucks in Form von kleinen Zeitsegmenten. Das Steuergerät verlangt in wiederkehrenden gleichen Zeitintervallen die Wiederholung bzw. Aufrechterhaltung des Tastendrucks. Erfolgt das nicht, wird dieser Zustand als nicht mehr betätigte Taste verstanden und die Infusion wird gestoppt.

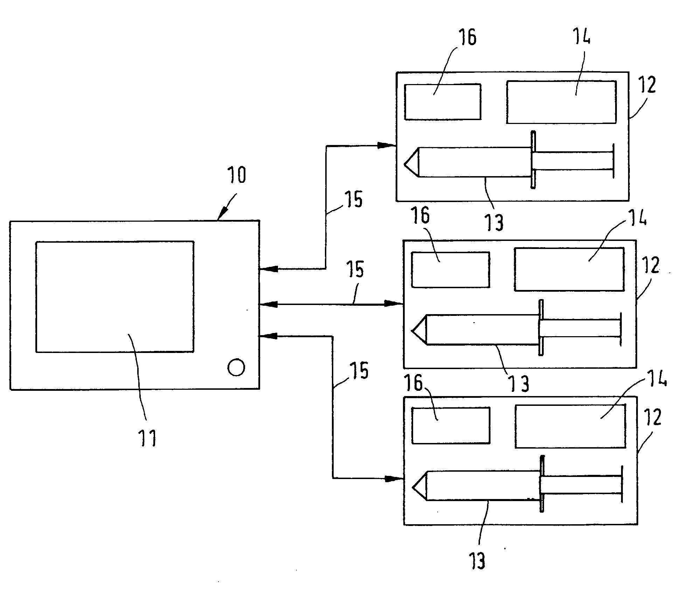

Im folgenden wird ein Ausführungsbeispiel der Erfindung unter Bezugnahme auf die einzige Figur der Zeichnung näher erläutert.

In der Zeichnung ist das Blockschaltbild einer Infusionsvorrichtung dargestellt.

Die Infusionsvorrichtung weist ein zentrales Steuergerät 10 auf, das aus einem Steuerrechner besteht und eine Bildschirmanzeige 11 sowie eine (nicht dargestellte) Eingabetastatur aufweist. Die Bildschirmanzeige 11 kann auch als Touchscreen ausgebildet sein und als Eingabevorrichtung benutzt werden.

Die Infusionsvorrichtung weist ferner ein Regime aus zahlreichen Infusionsgeräten 12 auf. Bei den Infusionsgeräten handelt es sich vorzugsweise um Infusionspumpen, insbesondere um Spritzenpumpen. Bei einer Spritzenpumpe wird der Inhalt einer Spritze 13 aus Spritzenzylinder und Kolben ausgedrückt und über einen Katheter zu dem Patienten übertragen. Das Ausdrücken der Spritze 13 erfolgt durch gesteuertes Vorschieben des Spritzenkolbens durch den Infusionspumpenantrieb.

Das Infusionsgerät 12 enthält ferner ein Steuerteil 14, bei dem es sich um einen Rechner handelt, welcher mit dem Steuergerät 10 kommuniziert. Die Datenkommunikation erfolgt jeweils über einen Datenkanal 15. Der Datenkanal 15 besteht aus einer elektrischen Leitung, insbesondere aus einer gegen Störbeeinflussungen gesicherten verdrehten Leitung oder einem Koaxialkabel. Er kann jedoch auch als Funkkanal ausgebildet sein. Sämtliche Infusionsgeräte 12, die an das Steuergerät 10 angeschlossen sind, sind demselben Patienten zugeordnet, d.h. ihre Flüssigkeitsleitungen sind über ein Schlauchsystem mit dem Patientenkörper verbunden.

Jedes Infusionsgerät 12 weist ferner eine Eingabe- und Anzeigevorrichtung 16 auf.

Wenn ein Infusionsgerät 12 an das Steuergerät 10 angeschlossen wurde, verlangt der zweikanalige Steuerrechner im Steuergerät 10 von dem Infusionsgerät 12 die Angabe der Identnummer. Die Identnummer wird dem Infusionsgerät 12 fabrikmäßig eingegeben. Es handelt sich um eine einzigartige Identnummer, die diesem speziellen Infusionsgerät 12 zugeordnet wird. Daraufhin sendet das Infusionsgerät die Identnummer an den Steuerrechner. Dieser überprüft die Identnummer darauf, ob sie bereits vorliegt. Wenn dies nicht der Fall ist, wird die Identnummer invertiert an das Infusionsgerät 12 zurückgeschickt. Erst nach Überprüfung der Korrektheit der invertierten Identnummer akzeptiert das Infusionsgerät 12 Fernsteuerbefehle vom Steuergerät 10.

Die Steuerbefehle und andere Daten werden mit einem Fehlererkennungscode übertragen. Dies geschieht durch Nutzung bekannter. Datensicherungsverfahren, beispielsweise durch Verwendung eines CRC-Polynoms als Sicherungswort. Hierzu werden die zweikanaligen Systeme im Steuerrechner und im Infusionsgerät in der Weise genutzt, dass ein Kanal das zu übertragende Datum zur Verfügung stellt, während der andere Kanal unabhängig vom ersten die zugehörige CRC-Sicherungssumme generiert.

Zusammen mit den Befehlen und Daten wird jeweils die Identnummer des adressierten Steuergerätes 10 übertragen.

Jedes Infusionsgerät 12 erwartet innerhalb einer vorgegebenen Fehlertoleranzzeit einen gültigen Kommunikationsablauf mit dem Steuergerät 10. Falls dieser aufgrund einer unterbrochenen Datenkommunikationsstrecke nicht stattfindet, stoppt das Infusionsgerät und geht in den für den Patienten sicheren Betriebszustand.

Das System ermöglicht zwei Betriebsarten, nämlich den Betriebsmodus "Fernsteuerung", bei dem Bedienungseingriffe an dem Infusionsgerät 12 akzeptiert und Verstellungen am Steuergerät 10 ignoriert werden, und den Betriebsmodus "Fernregelung", an dem Bedienungseingriffe an dem Infusionsgerät 12 ignoriert und Verstellungen am Steuergerät 10 akzeptiert werden.

Im Steuergerät 10 ist eine Taste als selbständige Taste oder als Touchscreen-Taste vorgesehen, bei deren Betätigung für die Dauer des Tastendrucks das Infusionsgerät eine vorgegebene einstellbare Infusionsrate auszuführen hat. Der Tastendruck wird in vorgegebenen kleinen Zeitsegmenten detektiert. Wird innerhalb eines dieser aufeinanderfolgenden Zeitintervalle eine Tastenbetätigung nicht erkannt, wird dies als nicht mehr betätigte Taste verstanden und die Infusion mit der genannten Infusionsrate beendet.

## Patentansprüche

1. Infusionsvorrichtung mit einem zentralen Steuergerät (10) und mehreren über Datenkanäle (15) mit dem Steuergerät kommunizierenden Infusionsgeräten (12),
**dadurch gekennzeichnet**,
dass jedes Infusionsgerät (12) eine unverwechselbare Identnummer enthält, dass das Steuergerät (10) über den Datenkanal (15) detektiert, wenn ein neues Infusionsgerät (12) angeschlossen wurde, und dann ein Abfragesignal an dieses Infusionsgerät liefert, dass das Infusionsgerät auf das Abfragesignal hin seine Identnummer an das Steuergerät (10) sendet, welches die Identnummer daraufhin überprüft, ob sie bereits vorliegt, dass das Steuergerät (10) ein der empfangenen Identnummer entsprechendes Signal an das Infusionsgerät (12) liefert und dass das Infusionsgerät (12) feststellt, ob das vom Steuergerät (10) gelieferte Signal zu der Identnummer paßt, und Befehle von dem Steuergerät (10) erst akzeptiert, wenn dies der Fall ist.

2. Infusionsvorrichtung nach Anspruch 1, dadurch gekennzeichnet, dass das der Identnummer entsprechende Signal aus der modifizierten Identnummer besteht.

3. Infusionsvorrichtung nach Anspruch 1 oder 2, dadurch gekennzeichnet, dass das Steuergerät (10) und der Steuerteil (14) des Infusionsgeräts (12) zweikanalig ausgeführt sind und dass jeweils ein Kanal das zu übertragende Datum zur Verfügung stellt, während der andere ein dem Datum entsprechendes Sicherungswort generiert.

4. Infusionsvorrichtung nach Anspruch 3, dadurch gekennzeichnet, dass die durch ein Sicherungswort gesicherten Daten durch die Identnummer des als Empfänger vorgesehenen Infusionsgerätes markiert sind, wobei das Infusionsgerät nur bei Übereinstimmung mit seiner Identnummer die Daten akzeptiert.

5. Infusionsvorrichtung nach einem der Ansprüche 1 bis 4, dadurch gekennzeichnet, dass das Infusionsgerät (12) innerhalb einer vorgegebenen Fehlertoleranzzeit einen gültigen Kommunikationsablauf mit dem Steuergerät (10) erwartet und bei Ausbleiben des Kommunikationsablaufs die Medikation stoppt.

6. Infusionsvorrichtung nach einem der Ansprüche 1 bis 5, dadurch gekennzeichnet, dass das Steuergerät (10) zwei Betriebsarten ausführen kann, wobei in einem Betriebsmodus "Fernsteuerung" Bedienungseingriffe am Infusionsgerät (12) akzeptiert und Verstellungen am Steuergerät (10) ignoriert werden, und in einem Betriebsmodus "Fernsteuerung" Bedienungseingriffe an dem Infusionsgerät (12) ignoriert und Verstellungen am Steuergerät (10) akzeptiert werden.

7. Infusionsvorrichtung nach einem der Ansprüche 1 bis 6, dadurch gekennzeichnet, dass das Drücken einer Taste in kleinen Zeitintervallen detektiert wird und dass ein Ausbleiben des Tastendrucks in einem Zeitintervall als nicht mehr betätigte Taste verstanden wird und zum Stoppen der Infusion führt.
